# EUROPÄISCHE PATENTSCHRIFT

(11) **EP 3 448 333 B1**
(45) Veröffentlichungstag und Bekanntmachung des Hinweises auf die Patenterteilung: **19.11.2025**
(21) Anmeldenummer: 17725854.8
(22) Anmeldetag: 28.04.2017
(51) Int. Cl.: A61F 6/14, A61F 6/18

(54) **INTRAUTERINPESSAR**
INTRAUTERINE DEVICE
DISPOSITIF INTRA-UTÉRIN

(30) Priorität: 28.04.2016 DE 102016107886
(43) Veröffentlichungstag der Anmeldung: 06.03.2019
(73) Patentinhaber: Gynerix GmbH, 66953 Pirmasens (DE)
(72) Erfinder: HUSCHMAND NIA, Hamid, 66953 Pirmasens (DE)
(74) Vertreter: Patentanwälte Bernhardt / Wolff Partnerschaft mbB
(86) Internationale Anmeldenummer: PCT/DE2017/100357
(87) Internationale Veröffentlichungsnummer: WO 2017/186235

(56) Entgegenhaltungen:
- WO-A1-2010/031899
- WO-A1-89/09038
- GB-A- 1 241 020
- US-A- 5 014 722

## Beschreibung

Die Erfindung betrifft ein Intrauterinpessar, das einen Trägerkörper und ein elastisch verformbares Mittel zum Halten des Intrauterinpessars in der Gebärmutter umfasst, das zumindest abschnittweise die Form eines Rings aufweist und sich in einer Einführposition zum Einführen des Intrauterinpessars in die Gebärmutter zumindest teilweise innerhalb des zumindest teilweise hohlen Trägerkörpers anordnen lässt und sich in eine Fixierposition bringen lässt, in der es von dem Trägerkörper vorsteht und das Intrauterinpessar unter Anlage an der Gebärmutterwand in der Gebärmutter halten kann.

Die Erfindung betrifft ferner ein Verfahren zum Einführen des Intrauterinpessars in die Gebärmutter.

Ein solches Intrauterinpessar ist aus der WO 89/09038 A1 bekannt, die ein Intrauterinpessar beschreibt, das elastisch verformbare Metalldrähte als Entfaltungsarme aufweist, die mittels mechanischer Verstellung eines dritten Arms zu einer Ringform entfaltet werden und dadurch das Intrauterinpessar in eine Verweilform gebracht wird in der die Entfaltungsarme an der Gebärmutterwand anliegen.

Ferner beschreibt die WO 2010/031899 A1 ein Intrauterinpessar, das einen ringförmigen Rahmen aufweist, und eine Vorrichtung zur Einführung des Intrauterinpessars in die Gebärmutter.

Aus der GB 1241020 A ist ein Intrauterinpessar bekannt, dass mittels eines fadenförmigen elastischen Haltemittels in einer Fixierposition in der Gebärmutter gehalten wird.

Durch Benutzung sind Intrauterinpessare unter der Bezeichnung "Kupferspiralen" oder "Hormonspiralen" bekannt und sind T- oder hufeisenförmig, damit sie im Inneren der Gebärmutter Halt finden. Problematisch ist, dass die Intrauterinpessare zur Anordnung in der Gebärmutter durch den verhältnismäßig engen Gebärmutterhalskanal hindurch geführt werden müssen und der Gebärmutterhals dazu normalerweise mit einer Kugelzange gefasst werden muss, um die Gebärmutter zu fixieren bzw. aufzurichten. Da die Intrauterinpessare dabei den Gebärmutterhals verformen, kann dies erhebliche Schmerzen verursachen.

Der Erfindung liegt die Aufgabe zugrunde, ein Intrauterinpessar der eingangs genannten Art zu schaffen, das sich schonender in die Gebärmutter einsetzen lässt.

Erfindungsgemäß wird diese Aufgabe dadurch gelöst, dass das Haltemittel in der Einführposition gegen eine Rückstellkraft elastisch verformt ist, sich das Haltemittel durch die Rückstellkraft hin zur Fixierposition bringen lässt und das Intrauterinpessar ein Betätigungsmittel aufweist, mittels dessen sich das Haltemittel gegen die Rückstellkraft in der Einführposition halten lässt.

Da das Haltemittel in der Einführposition nicht von dem Trägerkörper vorsteht und dementsprechend das Intrauterinpessar in der Einführposition wesentlich schmaler ist als in der Fixierposition, muss der Gebärmutterhals beim Einführen weniger verformt werden. Beim Einführen werden deshalb keine oder nur vergleichsweise geringe Schmerzen verursacht. Sobald das Intrauterinpessar an der vorgesehenen Stelle in der Gebärmutter angeordnet ist, wird das Haltemittel in die Fixierposition gebracht und hält das Intrauterinpessar in der Gebärmutter fest.

In einer Ausgestaltung der Erfindung steht das Haltemittel in der Fixierposition in radialer Richtung von dem Trägerkörper vor. Zweckmäßigerweise ist in dem Trägerkörper zumindest eine Öffnung gebildet, die zu einem Hohlraum des Trägerkörpers, in dem sich das Haltemittel in der Einführposition anordnen lässt, führt und durch die hindurch das Haltemittel von der Einführposition in die Fixierposition bewegbar ist. Vorzugsweise sind das Haltemittel und der Trägerkörper derart geformt, dass das Haltemittel in der Einführposition vollständig innerhalb des Trägerkörpers angeordnet ist.

In einer Ausführungsform der Erfindung, die ein besonders schonendes Einsetzen des Intrauterinpessars erlaubt, weist der Trägerkörper eine zylindrische oder eine im Wesentlichen zylindrische Form auf.

Zweckmäßigerweise umfasst der Trägerkörper einen Hohlzylinder, der bevorzugt auf einer seiner beiden Grundflächen geschlossen ist. In der Mantelfläche des Hohlzylinders ist vorzugsweise zumindest eine Öffnung gebildet, die zu dem Hohlraum des Hohlzylinders führt. Besonders bevorzugt sind in der Mantelfläche zumindest zwei gegenüberliegende Öffnungen gebildet. Durch die Öffnung bzw. die Öffnungen kann das Haltemittel aus dem Hohlraum heraus in die Halteposition und/oder aus der Halteposition heraus in die Fixierposition bewegt werden. Vorzugsweise ist die Öffnung bzw. sind die Öffnungen in der Mantelfläche im Abstand von der offenen Grundfläche des Hohlzylinders angeordnet. Zumindest in dem Endabschnitt des Hohlzylinders, an dem sich das offene Ende befindet, ist die Mantelfläche geschlossen und kann dort zumindest einen Abschnitt des Haltemittels aufnehmen.

In einer Ausgestaltung der Erfindung weist der Trägerkörper an seinem Ende, mit dem er zuerst in die Gebärmutter eingeführt wird, eine Form auf, die frei von Kanten ist, vorzugsweise eine runde Form, besonders bevorzugt eine halbkugelige Form. Zweckmäßigerweise ist die kantenfreie Form an dem Ende des Hohlzylinders gebildet, an dem die Grundfläche geschlossen ist.

Vorzugsweise weisen der Trägerkörper und/oder das Haltemittel eine glatte Oberfläche auf.

Zweckmäßigerweise weist das Haltemittel zumindest abschnittsweise die Form eines Strangs oder/und eines Rings auf. Vorzugsweise ist es durch einen Ring gebildet.

In einer besonders bevorzugten Ausgestaltung der Erfindung ist das Haltemittel in der Einführposition gegen eine Rückstellkraft elastisch verformt und lässt sich allein durch die Rückstellkraft oder/und unter Wirkung der Rückstellkraft hin zur, vorzugsweise in die, Fixierposition bringen. Das Haltemittel wird während des Einführens unter elastischer Verformung gegen die Rückstellkraft in der Einführposition gehalten und das Intrauterinpessar, sobald es in der vorgesehenen Position in der Gebärmutter angeordnet ist, losgelassen und bewegt sich durch Wirkung der Rückstellkraft von selbst hin zur bzw. in die Fixierposition.

Zweckmäßigerweise ist der Trägerkörper oder/und das Haltemittel aus einem, vorzugsweise biotoleranten oder bioinerten, Metall oder Kunststoff gebildet. Der Trägerkörper oder/und das Haltemittel können zur Ausbildung einer empfängnisverhütenden Wirkung zumindest teilweise aus Kupfer oder/und aus einem Stoff, vorzugsweise einem Kunststoff, der zur Abgabe von Kupferionen oder/und eines Hormons, insbesondere Steroidhormons, geeignet ist, gebildet sein.

In einer weiteren Ausgestaltung der Erfindung weist das Haltemittel in der Fixierposition zumindest in einem Abschnitt, der zu Abstützung an der Gebärmutterwand vorgesehen ist, eine Rundung auf, und ist zur Anordnung derart vorgesehen, dass es sich in einer Halteposition an der Gebärmutterwand mit einer Außenseite der Rundung abstützt. Vorteilhaft kann sich das Haltemittel damit auf einer verhältnismäßig großen Fläche der Gebärmutterwand und nicht nur punktuell abstützen. Das Risiko, dass die Gebärmutter in der Fixierposition oder beim Anordnen des Haltemittels in der Fixierposition durch das Intrauterinpessar verletzt wird, kann dadurch erheblich reduziert werden.

In einer Ausführungsform der Erfindung weist das Intrauterinpessar einen Faden auf, mittels dessen sich das Haltemittel gegen die Rückstellkraft in die Einführposition bringen und in ihr halten lässt.

Das Haltemittel ist zweckmäßigerweise zumindest an einem Ende des Hohlraums des Trägerkörpers an dem Trägerkörper befestigt. Das Betätigungsmittel greift an dem Haltemittel vorzugsweise im Abstand von der Stelle an, an der es an dem Trägerkörper gehalten ist, sodass es das Haltemittel zu dessen Anordnung in der Einführposition in den Hohlkörper hineinziehen kann.

Zweckmäßigerweise umfasst das Intrauterinpessar ein Mittel zum Blockieren des Betätigungsmittels in einer Blockierstellung, in der es das Haltemittel in der Einführposition hält. Vorzugsweise ist das Blockiermittel dazu vorgesehen, den Faden in der Blockierstellung einzuklemmen.

In einer weiteren Ausgestaltung der Erfindung umfasst das Intrauterinpessar ein Mittel zum Einführen des Intrauterinpessars in die Gebärmutter, das bzw. die mit dem Trägerköper lösbar verbindbar ist und vorzugsweise ein Rohr oder einen Stab umfasst. Das Betätigungsmittel, insbesondere der Faden, ist vorzugsweise durch das Rohr geführt oder entlang des Stabs angeordnet.

Zweckmäßigerweise weist das Einführmittel eine Feder auf, mittels derer sich der Trägerkörper von dem Einführmittel abdrücken lässt, um den Trägerkörper von dem Einführmittel zu lösen, wenn der Trägerkörper an der vorgesehenen Stelle in der Gebärmutter angeordnet ist. Zweckmäßigerweise ist das Einführmittel derart vorgesehen, dass sich der Trägerkörper mittels des Betätigungsmittels, vorzugsweise gegen die Kraft der Feder, an dem Einführmittel halten lässt.

In einer besonders bevorzugten Ausführungsform der Erfindung ist das Intrauterinpessar derart vorgesehen, dass sich durch das Betätigungsmittel gleichzeitig das Haltemittel in der Einführposition und der Trägerkörper an dem Einführmittel halten lässt und dass bei Lösen des Betätigungsmittels gleichzeitig das Haltemittel, insbesondere durch die Rückstellkraft, in die Fixierposition gebracht und der Trägerkörper von dem Einführmittel getrennt wird.

Vorzugsweise ist das Blockiermittel derart vorgesehen, dass es in der Blockierstellung gleichzeitig das Haltemittel in der Einführposition und das Einführmittel an dem Trägerkörper halten kann.

Die Erfindung wird nachfolgend anhand eines Ausführungsbeispiels und der beigefügten Zeichnungen, die sich auf das Ausführungsbeispiel beziehen, näher erläutert. Es zeigen
- Fig. 1 und 2: ein erfindungsgemäßes Intrauterinpessar in verschiedenen Ansichten,
- Fig. 3: ein Detail des Intrauterinpessars nach den Fig. 1 und 2, und
- Fig. 4: das erfindungsgemäße Intrauterinpessar nach den Fig. 1 bis 3 bei Anordnung in der Gebärmutter.

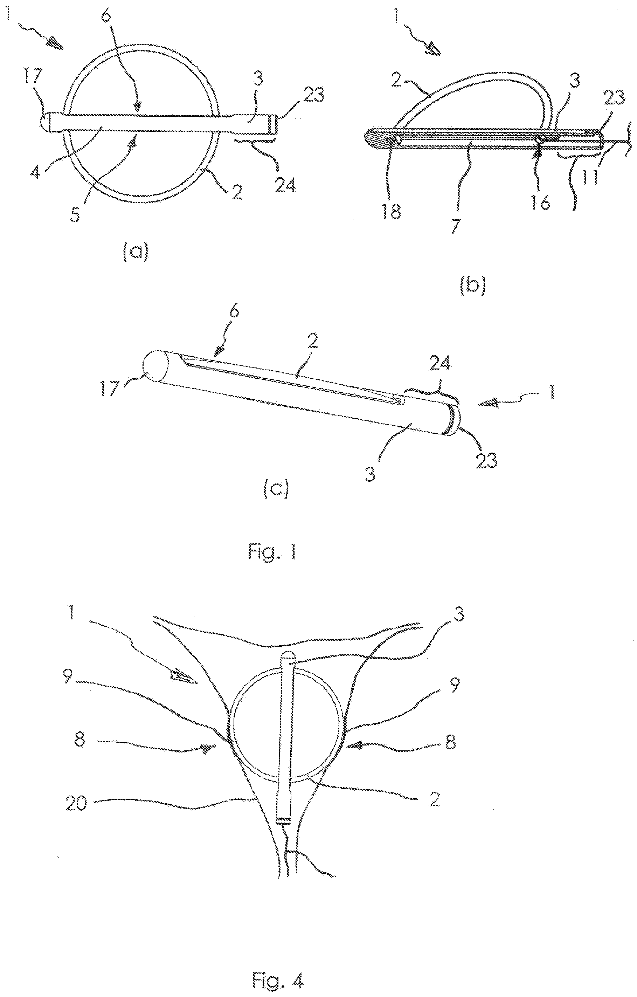

Ein in Fig. 1 gezeigtes erfindungsgemäßes Intrauterinpessar 1 umfasst einen Trägerkörper 3, der im Wesentlichen eine zylindrische Form aufweist und an dem ein Mittel 2 zum Halten des Intrauterinpessars 1 an der Gebärmutterwand angeordnet ist, das durch einen elastischen Ring 2 gebildet ist. Der Ring 2 ist auf einer Seite durch eine Bohrung 18, die in dem Trägerkörper 3 gebildet ist, geführt und wird dadurch an den Trägerkörper 3 gehalten. An einer gegenüberliegenden Seite ist der Ring 2 durch längliche Öffnungen 5,6 geführt, die durch eine Zylinderwand des Trägerkörpers 3 und zu einem Hohlraum 7 des Trägerkörpers 3 führen, der sich bis zu einem hinteren Ende 23 des Trägerkörpers 3 erstreckt. An dem Ende 23, d.h. an einer Grundfläche des abschnittsweise hohlzylindrischen Trägerkörpers 3, ist der Hohlraum 7 offen. Zwischen den Öffnungen 5,6 und dem Ende 23 besteht ein gewisser Abstand. In einem Abschnitt 24 zwischen dem Ende 23 und den Öffnungen 5,6 ist die Zylinderwand, die eine Mantelfläche des hohlzylindrischen Bereichs des Trägerkörpers 3 bildet, geschlossen.

Ein vorderes Ende 17 des Trägerkörpers 3, mit dem das Intrauterinpessar 1 zuerst in die Gebärmutter eingeführt wird, weist, wie insbesondere Fig. 1 zu entnehmen ist, eine runde Form auf, die frei von Kanten ist, um Verletzungen des Gebärmutterhalses und der Gebärmutter beim Einsetzen des Intrauterinpessars 1 zu vermeiden.

Der Trägerkörper 3 ist aus einem Metall, vorzugsweise aus Kupfer, oder/und einem Kunststoff gebildet. Der Ring 2 ist aus einem elastisch verformbaren Kunststoff gebildet. Der Kunststoff, aus dem der Trägerkörper 3 bzw. der Ring 2 gebildet ist, kann derart vorgesehen sein, dass er bei Anordnung in der Gebärmutter kontinuierlich Kupferionen oder/und Hormone, insbesondere Steroidhormone, freisetzt. Alternativ oder ergänzend dazu können der Trägerkörper 3 und/oder der Ring 2 zumindest abschnittsweise mit einem Stoff, vorzugsweise einem Kunststoff, belegt sein, der die Kupferionen oder Hormone freisetzt oder direkt mit einer Kupferschicht versehen oder/und dem Hormon beschichtet sein.

Fig. 1a und b zeigen den Intrauterinpessar 1 in einer Fixierposition, in der der Ring 2 nicht elastisch verformt ist und bezogen auf die zylindrische Form des Trägerkörpers 3 radial von dem Trägerkörper 3 wegsteht. Wie Fig. 4 zu entnehmen ist, legt sich der Ring 2 zum Halten des Intrauterinpessars 1 mit einem Abschnitt 8 in der Gebärmutter mit seiner runden Außenseite 9 an der Gebärmutterwand 20 an.

Fig. 1c zeigt das Intrauterinpessar 1 in einer Einführposition, in der es durch den Gebärmutterhals in die Gebärmutter eingeführt werden kann und der Ring 2 in den Hohlraum 7 des Trägerkörpers 3 hineingezogen ist. Das Intrauterinpessar 1 ist in der Einführposition verhältnismäßig schmal und lässt sich deshalb unter lediglich geringer Verformung des Gebärmutterhalses und dadurch unter Vermeidung von Schmerzen in die Gebärmutter einsetzen. Der Ring 2 kann dazu, wie in Fig. 1c dargestellt, so weit in den Hohlraum 7 gezogen werden kann, dass er nicht von dem Trägerkörper 3 vorsteht. Insbesondere lässt sich der Ring 2 zum Ende 23 des Trägerkörpers 3 hin in den Hohlraum 7, also auch in den genannten Abschnitt 24, in dem die Zylinderwand des Trägerkörpers 3 geschlossen ist, hineinziehen.

Wie insbesondere Fig. 1b zu entnehmen ist, ist der Ring 2 auf seiner Seite 16, die der Stelle, an der der Ring 2 durch die Bohrung 18 geführt ist, abgewandt ist, von einem Faden 11 umschlungen, mittels dessen sich der Ring 2 in den Hohlraum 7 hineinziehen und damit in die Einführposition bringen lässt. Der Faden 11 ist durch den Hohlraum 7 am hinteren Ende 23 aus dem Trägerkörper 3 herausgeführt.

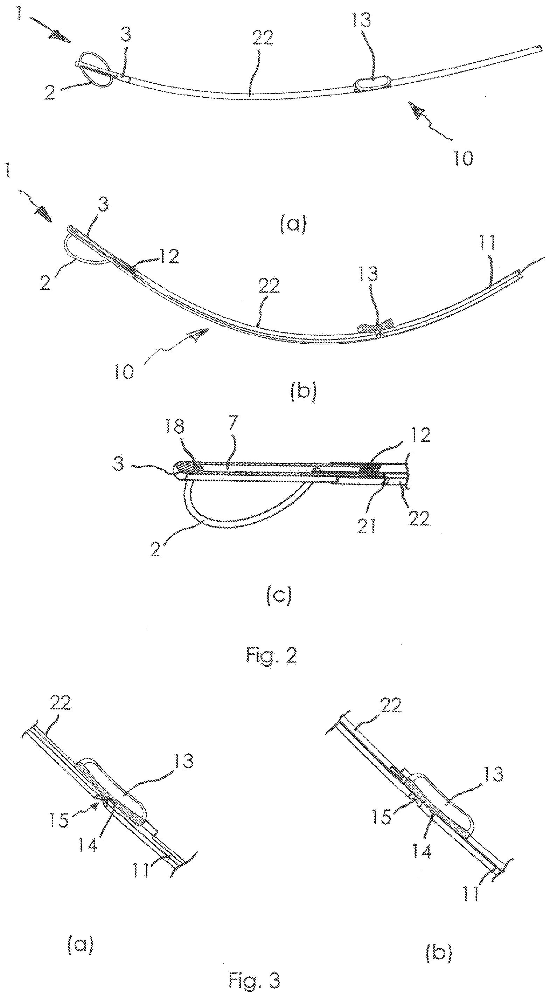

In Fig. 2 ist ein Einführmittel 10 dargestellt, auf das sich der Trägerkörper 3 kippstabil aufsetzen lässt. Das Einführmittel 10 umfasst ein Rohr 22, das an seinem einen Ende mit einer flanschartigen Formung versehen ist, in die der zylindrische Trägerkörper 3 eingesteckt werden kann. Wie insbesondere Fig. 2c zu entnehmen ist, ist auf der Innenseite des Rohrs 22 ein Absatz 21 gebildet, auf dem sich eine Feder 12 mit ihrem einem Ende abstützt. Mit dem anderen Ende liegt die Feder 12 gegen den Trägerkörper 3 an.

Das Einführmittel 10 weist ferner ein Mittel 13 zum Blockieren des Fadens 11 in dem Rohr 22 auf. Wie Fig. 3 zu entnehmen ist, kann das Blockiermittel 13 in einer in Fig. 3a gezeigten Blockierposition angeordnet werden, in der eine Rastnocke 14 in eine Rastkerbe 15 eingreift und den Faden 11 dadurch festsetzt. Fig. 3b zeigt das Blockiermittel 13 in einer Offenstellung, in der der Faden 11 in dem Rohr 22 frei beweglich ist. Zum Einführen des Intrauterinpessars 1 in die Gebärmutter wird der Trägerkörper 3 auf das Einführmittel 10 gesetzt und der Faden 11 so weit durch das Rohr 22 des Einführmittels 10 hindurchgeführt, dass er aus dem Rohrende, das dem Trägerkörper 3 abgewandt ist, herausragt.

Der Ring 2 wird in die Einführposition (Fig. 1c) gebracht, indem der Faden 11 am dem Trägerkörper 3 abgewandten Ende des Rohrs 22 gegriffen und in Zugspannung versetzt wird und dabei den Ring 2 in den Hohlraum 7 des Trägerkörpers 3 hineinzieht und gleichzeitig den Trägerkörper 3 unter Verformung der Feder 12 gegen das Einführmittel 10 zieht. Sobald der Ring 2 so weit in den Hohlraum 7 hineingezogen ist, dass er nicht mehr von dem Trägerkörper 3 vorsteht und die Feder 12 gespannt ist, wird das Blockiermittel 13 in die Blockierposition gebracht, in der der Faden 7 festgehalten wird. Dadurch werden der Ring 2 in dem Hohlraum 7 und die Feder 12 im gespannten Zustand gehalten. Nun wird der Trägerkörper 3 durch den Gebärmutterhals in die für es vorgesehene Position in der Gebärmutter gebracht. Die Lage des IUPs in der Gebärmutter kann anhand einer hier nicht gezeigten Skalierung auf der Außenseite des Rohres 22 abgelesen werden. Sobald die Position erreicht ist, wird das Blockiermittel 13 in die Offenstellung gebracht und dadurch der Faden 11 freigegeben. Durch die Rückstellkraft, die aufgrund der Verformung des Rings 2 wirkt, bewegt sich der Ring 2 von selbst in die in Fig. 4 gezeigte Fixierposition, in der er sich gegen die Gebärmutterwand 20 abstützt. Gleichzeitig drückt die Feder 12 den Trägerkörper 2 von dem Einführmittel 10 weg. Das Einführmittel 10 kann nun abgenommen und der Faden 11 so weit gekürzt werden, dass er nur noch wenige Millimeter aus dem Gebärmutterhals hinausragt.

## Patentansprüche

1. Intrauterinpessar, das einen Trägerkörper (3) und ein elastisch verformbares Mittel (2) zum Halten des Intrauterinpessars (1) in der Gebärmutter umfasst, das zumindest abschnittweise die Form eines Rings aufweist und sich in einer Einführposition zum Einführen des Intrauterinpessars (1) in die Gebärmutter zumindest teilweise innerhalb des zumindest teilweise hohlen Trägerkörpers (3) anordnen lässt und sich in eine Fixierposition bringen lässt, in der es von dem Trägerkörper (3) vorsteht und das Intrauterinpessar (1) unter Anlage an der Gebärmutterwand in der Gebärmutter halten kann,
**dadurch gekennzeichnet,**
**dass** das Haltemittel (2) in der Einführposition gegen eine Rückstellkraft elastisch verformt ist und sich durch die Rückstellkraft hin zur Fixierposition bringen lässt und das Intrauterinpessar (1) ein Betätigungsmittel (11) aufweist, mittels dessen sich das Haltemittel (2) gegen die Rückstellkraft in der Einführposition halten lässt.

2. Intrauterinpessar nach Anspruch 1,
**dadurch gekennzeichnet,**
**dass** sich das Haltemittel (2) durch die Rückstellkraft in die Fixierposition bringen lässt.

3. Intrauterinpessar nach Anspruch oder 2,
**dadurch gekennzeichnet,**
**dass** das Haltemittel (2) in der Einführposition vollständig innerhalb des Trägerkörpers (3) angeordnet ist.

4. Intrauterinpessar nach einem der Ansprüche 1 bis 3,
**dadurch gekennzeichnet,**
**dass** der Trägerkörper (3) im Wesentlichen die Form eines Zylinders aufweist und das Haltemittel (2) in der Fixierposition in radialer Richtung, vorzugsweise von der Mantelfläche (4) des Zylinders, vorsteht, wobei in der Mantelfläche (4) vorzugsweise zumindest eine Öffnung (5,6) gebildet ist, die zu einem Hohlraum (7) des Trägerkörpers (3), in dem sich das Haltemittel in der Einführposition anordnen lässt, führt und durch die hindurch das Haltemittel (2) von der Einführposition in die Fixierposition bewegbar ist.

5. Intrauterinpessar nach einem der Ansprüche 1 bis 4,
**dadurch gekennzeichnet,**
**dass** das Haltemittel (2) zumindest abschnittsweise die Form eines Strangs oder/und eines Rings aufweist.

6. Intrauterinpessar einem der Ansprüche 1 bis 5,
**dadurch gekennzeichnet,**
**dass** das Haltemittel (2) in der Fixierposition zumindest in einem Abschnitt (8), der zu Abstützung an der Gebärmutterwand vorgesehen ist, eine Rundung (9) aufweist, und zur Anordnung derart vorgesehen ist, dass er in einer Halteposition zur Abstützung an der Gebärmutterwand mit einer Außenseite der Rundung (9) vorgesehen ist.

7. Intrauterinpessar einem der Ansprüche 1 bis 6,
**dadurch gekennzeichnet,**
**dass** das Haltemittel (2) durch einen biegsamen Ring gebildet ist.

8. Intrauterinpessar einem der Ansprüche 1 bis 7,
**gekennzeichnet durch** ein Mittel (10) zum Einführen des Intrauterinpessars (1) in die Gebärmutter, das bzw. die mit dem Trägerköper (3) lösbar verbindbar ist und vorzugsweise ein Rohr (22) oder einen Stab umfasst, auf das bzw. den der Trägerkörper bevorzugt kippstabil aufsetzbar ist.

9. Intrauterinpessar nach Anspruch 8,
**dadurch gekennzeichnet,**
**dass** das Betätigungsmittel (11) einen Faden aufweist.

10. Intrauterinpessar nach Anspruch 9,
**dadurch gekennzeichnet,**
**dass** sich der Trägerkörper (3) mittels des Betätigungsmittels (11), vorzugsweise gegen die Kraft einer Feder (12) zum Wegdrücken des Trägerkörpers (3) von dem Einführmittel (10), an dem Einführmittel (10) halten lässt.

11. Intrauterinpessar nach einem der Ansprüche 1 bis 10,
**dadurch gekennzeichnet,**
**dass** der Trägerkörper (3) oder/und das Haltemittel (2) zur Ausbildung einer empfängnisverhütenden Wirkung zumindest teilweise aus Kupfer oder/und aus einem Stoff, vorzugsweise einem Kunststoff, der zur Abgabe von Kupferionen oder/und Hormonen geeignet ist, gebildet ist.

## Claims

1. Intrauterine pessary comprising a carrier body (3) and an elastically deformable means (2) for holding the intrauterine pessary (1) in the uterus, which means has at least in part the shape of a ring and, in an insertion position for insertion of the intrauterine pessary (1) into the uterus, can be arranged at least partially within the at least partially hollow carrier body (3) and can be brought into a fixing position, in which it protrudes from the carrier body (3) and can hold the intrauterine pessary (1) in the uterus by bearing on the uterine wall,
**characterized in that**
the holding means (2) in the insertion position is elastically deformed counter to a restoring force and, as a result of the restoring force, can be brought into the fixing position, and the intrauterine pessary (1) has an actuation means (11), by which the holding means (2) can be held in the insertion position counter to the restoring force.

2. Intrauterine pessary according to Claim 1,
**characterized in that**
the holding means (2) can be brought into the fixing position by the restoring force.

3. Intrauterine pessary according to Claim 1 or 2,
**characterized in that**
the holding means (2) in the insertion position is arranged completely within the carrier body (3).

4. Intrauterine pessary according to any one of Claims 1 to 3,
**characterized in that**
the carrier body (3) has substantially the shape of a cylinder, and the holding means (2) in the fixing position protrudes in the radial direction, preferably from the lateral surface (4) of the cylinder, wherein at least one opening (5, 6) is preferably formed in the lateral surface (4) and leads to a cavity (7) of the carrier body (3) in which the holding means can be arranged in the insertion position, and through which opening the holding means (2) can be moved from the insertion position into the fixing position.

5. Intrauterine pessary according to any one of Claims 1 to 4,
**characterized in that**
the holding means (2) has, at least in part, the shape of a strand and/or a ring.

6. Intrauterine pessary according to any one of Claims 1 to 5,
**characterized in that**
the holding means (2) in the fixing position has a rounded region (9), at least in a portion (8) provided to bear on the uterine wall, and is provided for arrangement in such a way that it is provided in a holding position for bearing on the uterine wall with an outer side of the rounded region (9).

7. Intrauterine pessary according to any one of Claims 1 to 6,
**characterized in that**
the holding means (2) is formed by a flexible ring.

8. Intrauterine pessary according to any one of Claims 1 to 7,
**characterized by** a means (10) for inserting the intrauterine pessary (1) into the uterus, which means is releasably connectable to the carrier body (3) and preferably comprises a tube (22) or a rod onto which the carrier body can be placed preferably in a manner stable against tilting.

9. Intrauterine pessary according to Claim 8,
**characterized in that**
the actuation means (11) has a thread.

10. Intrauterine pessary according to Claim 9,
**characterized in that**
the carrier body (3) can be held on the insertion means (10) by the actuation means (11), preferably counter to the force of a spring (12) for pushing the carrier body (3) away from the insertion means (10).

11. Intrauterine pessary according to any one of Claims 1 to 10,
**characterized in that**,
in order to provide a contraceptive effect, the carrier body (3) and/or the holding means (2) are formed at least partially of copper and/or of a substance, preferably a plastic, which is suitable for the delivery of copper ions and/or hormones.

## Revendications

1. Dispositif intra-utérin, qui comprend un corps support (3) et un moyen (2) élastiquement déformable destiné à retenir le dispositif intra-utérin (1) dans l'utérus, qui présente au moins par sections la forme d'un anneau et qui se laisse agencer, dans une position d'introduction destinée à introduire le dispositif intra-utérin (1) dans l'utérus, au moins partiellement à l'intérieur du corps support (3) au moins partiellement creux et se laisse amener dans une position de fixation dans laquelle il dépasse du corps support (3) et peut maintenir le dispositif intra-utérin (1) en butée contre la paroi utérine dans l'utérus, **caractérisé**
**en ce que** le moyen de retenue (2), dans la position d'introduction, est déformé élastiquement contre une force de rappel et se laisse amener par la force de rappel vers la position de fixation et le dispositif intra-utérin (1) présente un moyen d'actionnement (11) à l'aide duquel le moyen de retenue (2) se laisse maintenir contre la force de rappel dans la position d'introduction.

2. Dispositif intra-utérin selon la revendication 1, **caractérisé**
**en ce que** le moyen de retenue (2) se laisse amener dans la position de fixation par la force de rappel.

3. Dispositif intra-utérin selon la revendication 1 ou 2, **caractérisé**
**en ce que** le moyen de retenue (2), dans la position d'introduction, est agencé complètement à l'intérieur du corps support (3).

4. Dispositif intra-utérin selon l'une des revendications 1 à 3, **caractérisé**
**en ce que** le corps support (3) présente sensiblement la forme d'un cylindre et le moyen de retenue (2) dépasse dans la position de fixation, dans la direction radiale, de préférence de la surface d'enveloppe (4) du cylindre, au moins une ouverture (5, 6) étant de préférence formée dans la surface d'enveloppe (4), qui conduit vers un espace creux (7) du corps support (3), dans lequel le moyen de fixation se laisse agencer dans la position d'introduction, et à travers laquelle le moyen de retenue (2) peut être déplacé de la position d'introduction dans la position de fixation.

5. Dispositif intra-utérin selon l'une des revendications 1 à 4, **caractérisé**
**en ce que** le moyen de retenue (2) présente au moins par sections la forme d'un brin ou/et d'un anneau.

6. Dispositif intra-utérin selon l'une des revendications 1 à 5, **caractérisé en ce que** le moyen de retenue (2), dans la position de fixation, au moins dans une section (8), qui est prévue pour l'appui contre la paroi utérine, présente une courbure (9) et est prévu, pour l'agencement, de telle sorte que dans une position de retenue, il est prévu pour l'appui contre la paroi utérine avec un côté externe de la courbure (9).

7. Dispositif intra-utérin selon l'une des revendications 1 à 6, **caractérisé en ce que** le moyen de retenue (2) est formé par un anneau souple.

8. Dispositif intra-utérin selon l'une des revendications 1 à 7, **caractérisé par** un moyen (10) destiné à introduire le dispositif intra-utérin (1) dans l'utérus, qui peut être relié de manière amovible au corps support (3) et qui comprend de préférence un tube (22) ou une tige sur lequel/laquelle le corps support peut être placé de préférence de manière stable contre une inclinaison.

9. Dispositif intra-utérin selon la revendication 8, **caractérisé en ce que** le moyen d'actionnement (11) présente un fil.

10. Dispositif intra-utérin selon la revendication 9, **caractérisé en ce que** le corps support (3) se laisse maintenir sur le moyen d'introduction (10) à l'aide du moyen d'actionnement (11), de préférence contre la force d'un ressort (12) destiné à repousser le corps support (3) du moyen d'introduction (10).

11. Dispositif intra-utérin selon l'une des revendications 1 à 10, **caractérisé en ce que** le corps support (3) et/ou le moyen de retenue (2) est/sont formé(s) au moins partiellement à partir de cuivre et/ou à partir d'une substance, de préférence un matériau synthétique, qui convient pour la libération d'ions de cuivre et/ou d'hormones, pour la réalisation d'un effet de contraception.
